Europäisches Patentamt

European Patent Office

Office européen des brevets

(19)

(11) **EP 1 247 096 B1**

(12) **FASCICULE DE BREVET EUROPEEN**

(45) Date de publication et mention
de la délivrance du brevet:
**30.03.2005 Bulletin 2005/13**

(21) Numéro de dépôt: **01903931.2**

(22) Date de dépôt: **12.01.2001**

(51) Int Cl.7: **G01N 33/544**

(86) Numéro de dépôt international:
**PCT/FR2001/000095**

(87) Numéro de publication internationale:
**WO 2001/051927 (19.07.2001 Gazette 2001/29)**

(54) **PROCEDE D'IMMOBILISATION DE REACTIF(S) AFFIN(S) SUR PHASE SOLIDE HYDROPHOBE**

VERFAHREN ZUR IMMOBILISIERUNG VON AFFINEN REAGENTIEN AN HYDROPHOBEN
FESTPHASEN

METHOD FOR IMMOBILISATION OF (AN) AFFINITY REAGENT(S) ON A HYDROPHOBIC SOLID
PHASE

(84) Etats contractants désignés:
**AT BE CH CY DE DK ES FI FR GB GR IE IT LI LU
MC NL PT SE TR**

(30) Priorité: **13.01.2000 FR 0000376**

(43) Date de publication de la demande:
**09.10.2002 Bulletin 2002/41**

(73) Titulaire: **Bio-Rad Pasteur
92430 Marnes-la-Coquette (FR)**

(72) Inventeurs:
• **BABIN, Fabienne
F-78180 Montigny le Bretonneux (FR)**
• **HAMON, Laurence
F-75017 Paris (FR)**
• **RIEUNIER, François
F-78390 Bois d'Arcy (FR)**

(74) Mandataire: **Gillard, Marie-Louise et al
Cabinet Beau de Loménie,
158, rue de l'Université
75007 Paris (FR)**

(56) Documents cités:
**US-A- 5 437 983**

• **SITUMORANG M., GOODING J.J., HIBBERT D.B.:
"Immobilisation of enzyme throughout a
polytyramine matrix: a versatile procedure for
fabricating biosensors" ANALYTICA CHIMICA
ACTA, vol. 394, 9 août 1999 (1999-08-09), pages
211-223, XP000946857 cité dans la demande**
• **BASTOS-GONZALEZ, HIDALGO-ALVAREZ ET
AL.: "Carboxylated Latexes for Covalent
Coupling Antibodies, I" JOURNAL OF COLLOID
AND INTERFACE SCIENCE, vol. 176, - 1995
pages 232-239, XP000952060 cité dans la
demande**
• **SÜDI P., DALA E., SZAJANI B.: "Preparation,
Characterization, and application of a Novel
Immobilized Carboxypeptidase B" APPLIED
BIOCHEMISTRY AND BIOTECHNOLOGY, vol.
22, - 1989 pages 31-43, XP000952096**
• **DAGENAIS P., DESPREZ B., ALBERT J.,
ESCHER E.: "Direct Covalent Attachment of
Small Peptide Antigens to Enzyme-Linked
Immunosorbent Assay Plates Using Radiation
and Carbodiimide Activation" ANALYTICAL
BIOCHEMISTRY, vol. 222, 1994, pages 149-155,
XP002149598**

Il est rappelé que: Dans un délai de neuf mois à compter de la date de publication de la mention de la délivrance du
brevet européen, toute personne peut faire opposition au brevet européen délivré, auprès de l'Office européen des
brevets. L'opposition doit être formée par écrit et motivée. Elle n'est réputée formée qu'après paiement de la taxe
d'opposition. (Art. 99(1) Convention sur le brevet européen).

**Description**

L'invention concerne un procédé d'immobilisation de réactif(s) affin(s) sur une phase solide hydrophobe utilisable en essais biologiques de détection d'analyte.

On connaît depuis des décennies des essais d'analyse biologique qui mettent en oeuvre des réactifs ayant une affinité l'un pour l'autre. On désignera ci-après ces derniers par le terme "réactifs affins" ou "réactifs de paire d'affinité". Ainsi on sait rechercher et détecter un membre d'une paire d'affinité au moyen de l'autre. Parmi les essais biologiques mettant en oeuvre une réaction d'affinité entre des réactifs affins, ou "essais d'affinité", on trouve les analyses enzymatiques mettant en oeuvre, par exemple, une enzyme et son substrat, les immunoessais qualitatifs ou quantitatifs, initiés par les travaux pionniers de Berson et Yalow (1959) et mettant en jeu la réaction d'un anticorps avec l'antigène ou l'haptène correspondant, plus récemment les essais d'hybridation d'acide nucléique mettant en oeuvre un oligo- ou poly-nucléotide cible avec une sonde nucléotidique complémentaire, capable de s'hybrider spécifiquement avec lui, etc.

Depuis les années 1960, on a cherché à mettre en oeuvre, dans les essais d'affinité nécessitant une séparation des complexes libres et des complexes liés obtenus, des phases solides (ou supports solides) réactives, afin de simplifier cette étape de séparation.

L'immobilisation des réactifs affins sur une phase solide peut être pratiquée par couplage covalent, par exemple à l'aide du glutaraldéhyde. On sait aussi, depuis les travaux de Catt et Tregear en 1967, réaliser l'immobilisation d'un réactif affin sur une phase solide par simple adsorption passive.

L'immobilisation par adsorption passive présente l'avantage de la simplicité, mais elle peut entraîner le relargage dans un milieu liquide du réactif non convenablement immobilisé.

Les couplages covalents sur phase solide présentent en général l'avantage de conduire à une plus grande stabilité du réactif final, mais aussi ils permettent d'immobiliser davantage de réactif affin sur un support solide. Nombreux sont les modes et agents de couplage covalent sur phase solide qui sont actuellement disponibles : on citera, à titre d'exemples non limitatifs, les couplages au glutaraldéhyde, au bromure de cyanogène, aux carbodiimides, en présence ou non de coactivateur, tel que la DMAP (diméthyl-aminopyridine), le HOBt (1-hydroxybenzotriazole), le N-hydroxysuccinimide, le s-NHS (sulfo-N-hydroxysuccinimide) bien connus de l'homme du métier.

Les différents protocoles d'immobilisation de réactif affin sur une phase solide par couplage covalent se réalisent soit en une étape, soit en deux, voire trois étapes. Lors des couplages en une étape, tous les ingrédients sont mis en contact les uns avec les autres. Les couplages en deux étapes font en général intervenir une première étape où le support solide est activé par un agent dit "agent d'activation", puis lavé afin que soit éliminé tout excès d'agent d'activation qui n'a pas réagi, pour être enfin mis en présence du réactif affin, ce qui permet de réaliser, dans une seconde étape, le couplage proprement dit.

De nombreuses phases ou supports solides sont connus et utilisés : les phases solides hydrophiles (comme, par exemple, le Sephadex[R] commercialisé par la société Pharmacia) et les phases solides hydrophobes (comme, par exemple, le polypropylène, le polystyrène, les latex ...). Ces dernières sont généralement rendues réactives par l'intermédiaire de fonctions qui leur sont préalablement greffées, telles que les fonctions amine, carboxyle, tosyle, aldéhyde, hydroxyle, thiol, chlorométhyle, hydrazide, etc.

Différentes techniques ont été proposées pour coupler de façon covalente des réactifs affins à des phases solides hydrophobes fonctionnalisées par un groupe carboxyle : diverses combinaisons de tampon et agent d'activation sont utilisées comme la combinaison du MES (acide 2-[N-morpholino]-éthane-sulfonique) avec un carbodiimide. On citera, à titre d'exemple, le couplage covalent d'anticorps à des latex carboxyliques, en présence de MES et d'EDC (1-éthyl-3-[3-diméthylaminopropyl]-carbodiimide), décrit par D. Bastos-Gonzalez et al., J. of Colloid and Interface Science, 176, 232-239 (1995) ou par C. Bieniarz et al., Bioconjugate Chem., 1996, 7, 88-95.

Toutefois, on sait depuis longtemps que la combinaison de tampon phosphate et carbodiimide - comme agent d'activation - est à éviter, car les carbodiimides ont l'inconvénient d'activer aussi les phosphates et de perdre ainsi une grande partie de leur réactivité. Il s'ensuit donc à la fois une insuffisance d'efficacité de couplage covalent, une grande proportion d'adsorption passive et, par voie de conséquence, une instabilité des produits obtenus (Wong, S. dans "Chemistry of protein conjugation and crosslinking", chapitre 6, page 196, (1991) et M.A. Gilles et al., Analytical Biochemistry 184, 244-248, (1990).

Bangs (Bangs Laboratories Inc., Tech. Note # 13c, Covalent coupling protocols, page 3) a proposé un protocole d'activation des phases solides hydrophobes fonctionnalisées par un groupe carboxyle à l'aide du carbodiimide EDC en solution aqueuse, mais les résultats obtenus sont loin d'être satisfaisants sur le plan de l'efficacité de couplage. En effet, le couplage n'est pas quantitatif et la part d'adsorption passive est importante par rapport au couplage covalent (voir exemple 1 ci-après).

D'une manière générale, avec toutes les techniques de l'art antérieur visant à immobiliser par couplage covalent un réactif affin sur une phase solide hydrophobe fonctionnalisée par un groupe carboxyle, on observe, en même temps que le couplage covalent proprement dit entre le réactif affin et la phase solide, la coexistence indésirée d'une fixation importante par adsorption passive dudit réactif affin sur la ladite phase solide. En d'autres termes, ces techniques ont l'inconvénient de ne pas pouvoir permettre la fixation covalente maximale souhaitée. L'adsorption passive ne pouvant pas être maîtrisée au profit du couplage covalent, il s'ensuit que ces techniques ne permettent pas d'optimiser au mieux la fixation covalente du réactif affin, ni donc d'obtenir des couplages covalents reproductibles. En conséquence, elles donnent naissance à des produits instables dans le temps.

Il existe donc un réel besoin de disposer d'une méthode d'immobilisation de réactif affin sur phase solide hydrophobe fonctionnalisée par un groupe carboxyle qui permette de maîtriser et d'optimiser de manière reproductible le caractère covalent du couplage tout en éliminant au maximum la possibilité d'adsorption passive simultanée.

On a maintenant trouvé, de façon surprenante, qu'il était possible de maîtriser et d'optimiser de manière reproductible le caractère covalent d'une réaction d'immobilisation de réactif affin sur une phase solide hydrophobe fonctionnalisée par un groupe carboxyle à l'aide d'une combinaison d'un carbodiimide et d'un tampon phosphate, comme agent d'activation.

L'invention a donc pour objet un procédé d'immobilisation d'un réactif affin sur une phase solide hydrophobe fonctionnalisée par un groupe carboxyle, ledit procédé comprenant une étape d'activation de ladite phase solide et une étape de couplage du réactif affin sur la phase solide et étant caractérisé en ce que l'on utilise, lors de l'étape d'activation de ladite phase solide, une combinaison d'un carbodiimide et d'un tampon phosphate en milieu acide, en présence d'un coactivateur, et en ce que l'étape de couplage du réactif affin est réalisée en milieu basique.

On peut utiliser aux fins de l'invention n'importe quel carbodiimide utilisé dans ce domaine à titre d'agent d'activation de fonction carboxyle, ainsi que dans le domaine de la synthèse peptidique.

Des exemples de carbodiimides sont décrits notamment par Lundblad, R.L. et al., Chemical Reagents for Protein Modification, vol. 2. Chap- 4, CRC Press, Boca Raton, F.L. et Marion Mikolajczyk et al., Tetrahedron vol. 37, pp 233-284 (1981).

Parmi les carbodiimides utilisables comme activateur dans le cadre de l'invention, on peut citer notamment le CMC (N-cyclohexyl-N'-(2-morpholinoéthyl)-carbodiimide méthyl-p-toluène sulfonate), l'EDC (1-éthyl-3-[3-diméthylaminopropyl]carbodiimide), le CMC étant particulièrement préféré.

Le carbodiimide doit être utilisé en excès par rapport à la fonction COOH. Avantageusement, on utilise une quantité de 20 à 50 équivalents molaires par fonction COOH.

Par "tampon phosphate" on entend, dans le cadre de l'invention, tout tampon phosphate classique (de sodium et/ou potassium) utilisé à une concentration allant généralement de 30 à 200 mM, le tampon phosphate 50 mM étant plus particulièrement préféré .

On peut utiliser n'importe quel coactivateur utilisé dans ce domaine. Des exemples de coactivateurs sont décrits notamment par Staros, J. V. Biochemistry 21, 3950-3955 (1982) ; O'Sullivan, M.J. et al., Anal. Biochem. 100, 100-108 (1979) et Abdella, P.M., et al. Biochem. Biophys. Res. Commun. 87, 734-742 (1979).

Parmi les coactivateurs utilisables selon l'invention, on peut utiliser notamment le s-NHS (sulfo N-hydroxysuccinimide), l'HOBt (1-hydroxybenzotriazole), le N-hydroxysuccinimide, le s-NHS (sulfo N-hydroxysuccinimide) étant particulièrement préféré.

Comme le carbodiimide, le coactivateur doit être utilisé en excès par rapport à la fonction COOH. Avantageusement, on utilise une quantité de 3 à 10 équivalents molaires par fonction COOH.

Par "milieu acide" dans l'étape d'activation du procédé de l'invention, on entend tout milieu ayant un pH allant d'environ 4 à environ 6,5, le pH 6 étant plus particulièrement préféré, le caractère acide dudit milieu étant conféré par le tampon phosphate.

Par "milieu basique" dans l'étape de couplage du procédé de l'invention, on désigne un milieu ayant un pH allant d'environ 7,2 à environ 10,5, un milieu contenant 50 % d'un tampon de pH 8,5 étant plus particulièrement préféré.

Le caractère basique du milieu est obtenu par l'utilisation de tampons classiques appropriés, par exemple un tampon borate ou un mélange tampon phosphate/tampon borate.

Ainsi, dans un mode de réalisation préféré de l'invention, on met en oeuvre, lors de l'étape d'activation de la phase solide, une combinaison de 20 à 50 équivalents molaires par fonction COOH de CMC, en tampon phosphate 30-200 mM, en présence de 3 à 10 équivalents molaires par fonction COOH de coactivateur sulfo-N-hydroxysuccinimide, en milieu acide ayant un pH allant d'environ 4 à environ 6,5 et l'on réalise le couplage en milieu basique ayant un pH allant d'environ 7,2 à environ 10,5.

Dans un mode de réalisation particulièrement préféré de l'invention, on met en oeuvre, lors de l'étape d'activation de la phase solide, une combinaison de 30 équivalents molaires par fonction COOH de CMC, en tampon phosphate $KH_2PO_4$ 50 mM, en présence de 5 équivalents molaires par fonction COOH de coactivateur sulfo-N-hydroxysuccinimide, à pH 6, et l'on réalise le couplage dans un milieu contenant 50 % d'un tampon borate de pH 8,5.

La combinaison judicieuse des paramètres d'activation : carbodiimide, tampon phosphate, coactivateur, et pH acide associée aux conditions de couplage décrites ci-dessus, est essentielle pour la mise en oeuvre du procédé de l'invention.

Les réactifs affins que l'on peut immobiliser dans le cadre de l'invention sont tous les composés qui ont une fonction amine ou peuvent en être artificiellement pourvus. On citera, à titre de tels réactifs affins, les protéines, les peptides, les immunoglobulines, les antigènes, les haptènes, les anticorps, les enzymes, les substrats enzymatiques, les oligonucléotides, les polynucléotides, etc., ainsi que tout autre réactif biologique connu de l'homme du métier.

Par "phase solide hydrophobe", on désigne dans la présente description les phases solides constituées de polymères hydrophobes couramment utilisés dans ce domaine, par exemple les polypropylènes, les polymères vinyl-laromatiques, tels que les polystyrènes, notamment les latex de ces polymères. Ces phases solides sont fonctionnalisées par un groupe carboxyle selon les techniques bien connues de l'homme du métier. A cet effet, on pourra se référer par exemple à l'article de Ottewill R.H. et al., dans Kolloid Zu Z Polymere 215:161-166(1967).

Parmi les phases solides hydrophobes fonctionnalisées par un groupe carboxyle utilisables selon l'invention, on citera les particules de latex, par exemple celles connues sous la marque Estapor$^R$ (Prolabo, France), les particules magnétiques Dynabeads$^R$ de la société Dynal, les microsphères Polybead$^R$ de la société Polysciences, Inc., et leurs équivalents, etc.

Les complexes solides réactifs susceptibles d'être obtenus par le procédé selon l'invention sont notamment les complexes phase solide-antigène, les complexes phase solide-haptène, les complexes phase solide-anticorps, etc., utilisables en immunoessais, ou les complexes phase solide-oligo- ou poly-nucléotide, utilisables en essais d'hybridation nucléique, d'amplification, etc. les complexes phase solide-enzyme ou les complexes phase solide-substrat enzymatique, etc.

Ces complexes peuvent être utilisés dans des "trousses" ou "nécessaires" ou "kits" pour essais biologiques, comme par exemple, et sans restriction, les immunoessais, les essais d'hybridation d'acide nucléique, les essais d'amplification d'acide nucléique, les essais enzymatiques, etc. connus de l'homme du métier, qu'ils soient qualitatifs ou quantitatifs.

L'invention sera mieux comprise à l'aide des exemples qui suivent et qui ne doivent aucunement être compris comme restreignant la portée de l'invention, mais sont donnés à titre simplement illustratif.


EXEMPLE 1 : COUPLAGE D'UN PEPTIDE SUR DES BILLES CARBOXYLIQUES


a) Réactifs


a1) Billes carboxyliques


Comme phase solide hydrophobe à groupe carboxyle, on a utilisé des billes de latex carboxyliques magnétiques produites par la société Prolabo, France (référence Estapor MI-070/60). Elles sont constituées de particules polydisperses de polystyrène et d'oxyde de fer, fonctionnalisées par des fonctions COOH. La phase solide utilisée a les caractéristiques suivantes : diamètre moyen (0,8 μm), pourcentage de fer (62 %), taux de fonctionnalisation (150 μeq COOH/g), présentée en suspension aqueuse à 0,1g/ml.

Toutes les étapes de lavage des billes sont réalisées de la façon suivante :

Dans chaque expérience, les billes magnétiques présentes dans les tubes à essai sont séparées des solutions à l'aide d'un support aimanté. Les surnageants sont éliminés à l'aide d'une pipette, les billes magnétiques étant retenues dans lesdits tubes à l'aide du support aimanté. Après chaque addition d'une nouvelle solution ou d'un nouveau tampon, les billes sont remises en suspension par vortexage pendant environ 10 secondes.

Un lavage comprend l'addition de la solution de lavage, la remise en suspension des billes et l'élimination de cette solution par aimantation.


a2) Peptide


Dans cet exemple, on a utilisé le peptide de 17 acides aminés ayant la séquence suivante: KGSYSVDHFRWGRPVSG-NH2.

Ce peptide a été préparé selon le mode opératoire décrit par E. Atherton and R.L. Sheppard, dans "Solid phase peptide synthesis, a practical approach", IRL PRESS, (1989), Oxford University Press, pp. 25-34.

La solution de tampon phosphate utilisée était une solution aqueuse de $KH_2PO_4$ 50 mM, à pH 6.

Toutes les opérations ont été réalisées à température ambiante, soit à 19-24°C.

b) immobilisation du peptide sur les billes carboxyliques

b1) Couplage covalent du peptide sur les billes carboxyliques

*Etape 1 : lavage des billes carboxyliques :*

Dans un tube à essai, 50 µl de billes de latex carboxyliques magnétiques sont lavées 2 fois avec 750 µl de NaOH 0,1 mM, pH 9, une fois avec 750 µl d'eau bi-distillée et, enfin, une dernière fois avec 750 µl de tampon phosphate.

*Etape 2 : activation des billes carboxyliques :*

Au culot de billes lavées obtenues à l'étape 1, on ajoute 250 µl de tampon phosphate, 200 µl (30 eq/ fonction COOH) d'une solution aqueuse de CMC [[N-cyclohexyl-N'-(2-morpholinoéthyl)-carbodiimide méthyl-p-toluène sulfonate] (Fluka)] et 50 µl (5 eq/COOH) d'une solution aqueuse de s-NHS [(sulfo-N-hydroxysuccinimide) (Pierce)].
On laisse incuber l'ensemble réactionnel pendant 1 heure à température ambiante, sous agitation. Les billes sont ensuite lavées avec 500 µl de tampon phosphate.

*Etape 3 : couplage d'un peptide sur les billes*

Au culot de billes activées obtenues à l'étape 2, on ajoute 250 µl de tampon phosphate, et 250 µl d'une solution de tampon borate 37,5 mM/NaCl 50 mM, pH 8,5 contenant 0,25 eq (par rapport aux fonctions COOH) du peptide décrit à la section a2).
On laisse incuber l'ensemble réactionnel pendant 1 heure à température ambiante, sous agitation. Les billes sont séparées par aimantation et le surnageant est conservé pour un dosage par HPLC (cf. section c) ci-après). Après lavage, les billes sont conservées dans du tampon PBS pH 7,4 ou tout autre tampon équivalent adapté.

b2) Adsorption passive du peptide sur les billes carboxyliques

Un couplage de type "passif - encore appelé "adsorption passive" - est réalisé en utilisant exactement les même réactifs que ci-dessus, mais en omettant les agents d'activation et de couplage CMC et s-NHS. Après couplage de type « passif », les billes sont séparées par aimantation et le surnageant conservé pour dosage HPLC (cf.section c)).

c) Calcul du rendement de couplage covalent et évaluation du couplage passif par HPLC

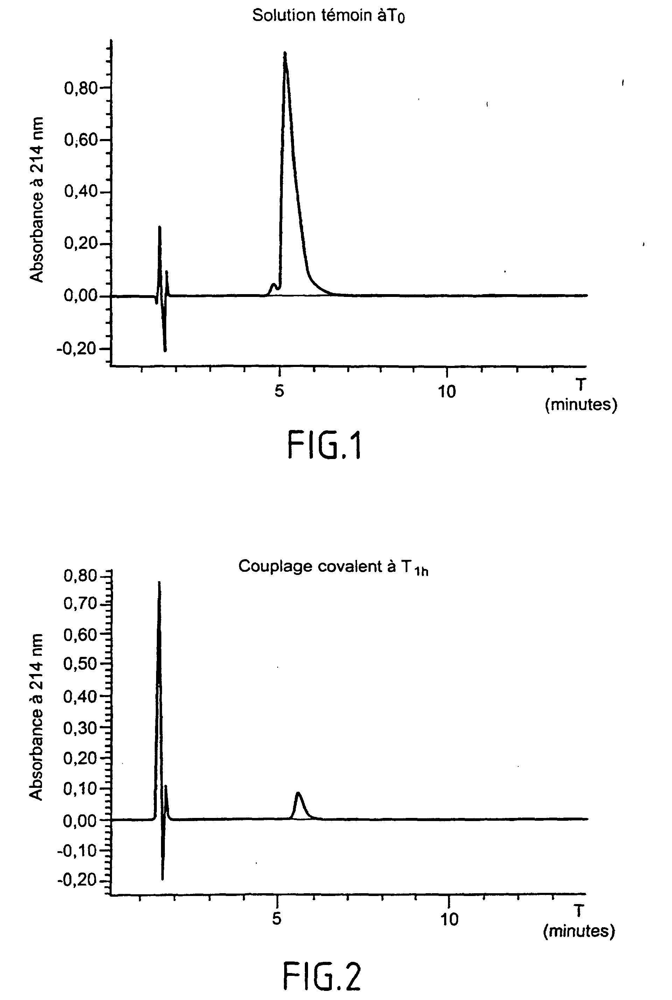

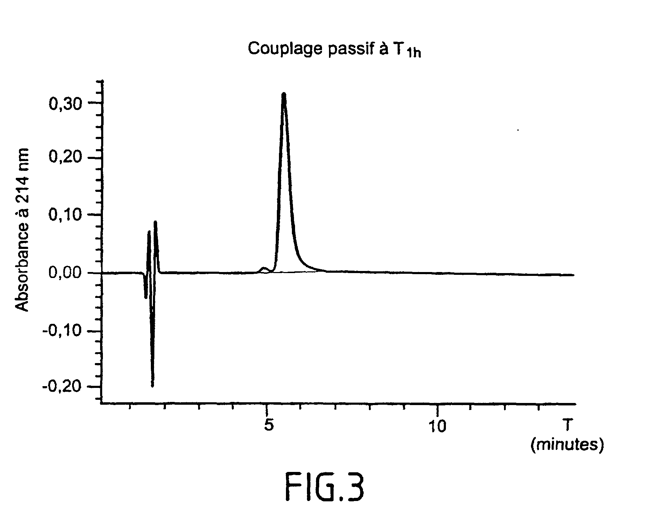

Les couplages sont suivis par chromatographie liquide à haute performance (HPLC) en phase inverse sur un appareil (Waters, par exemple) avec une phase stationnaire apolaire (C18) et une phase mobile polaire (gradient d'acétonitrile-TFA à 0,08 % dans l'eau -TFA à 0,1%).
A la fin de chaque couplage (covalent ou passif), on injecte dans l'appareil HPLC un volume de 30 µl du surnageant obtenu à la section b1) ou b2).
En outre, un volume identique de "solution témoin" (c'est-à-dire de la même solution de peptide contenu dans les 250 µl de tampon borate 37,5 mM/NaCl 50 mM, pH 8,5 et les 250 µl de tampon phosphate, utilisée lors des couplages, mais non soumise à couplage avec les billes magnétiques) est aussi injecté dans l'appareil HPLC.
Les chromatogrammes HPLC, obtenus en mesurant l'absorbance à 214 nm en fonction du temps T exprimé en minutes, sont représentés sur les figures 1 à 3. Le chromatogramme de la figure 1 montre le pic obtenu avec une solution témoin (essai sans couplage avec les billes magnétiques) à $T_0$. Elle sert de référence en indiquant la quantité totale de peptide introduite.
Le chromatogramme de la figure 2 montre le pic obtenu avec un surnageant prélevé à $T_{0+1\ heure}$ à la fin du couplage covalent décrit à la section b1).
Le chromatogramme de la figure 3 montre le pic obtenu avec un surnageant prélevé à $T_{0+1\ heure}$ à la fin de l'adsorption passive décrite à la section b2).
Pour chaque chromatogramme, l'aire du pic est intégrée par un logiciel (logiciel Millenium, par exemple). On mesure ainsi l'aire A0 pour la solution témoin, l'aire A1 pour le couplage covalent et l'aire A2 pour le couplage de "type passif".
Le rendement de couplage est ainsi déterminé par un dosage en retour, connu de l'homme du métier, par la formule suivante :

$$\% \text{ de couplage covalent} = \frac{(A0 - A1)}{A0} \times 100$$

$$\text{\% de couplage "passif"} = \frac{(A0 - A2)}{A0} \times 100$$

d) Comparaison du couplage selon l'invention et de couplages selon l'art antérieur

L'étude a été réalisée sur 3 protocoles de l'art antérieur et sur le protocole selon l'invention. Un couplage dans des conditions "passives", en omettant l'agent de couplage et l'agent d'activation, a été réalisé en parallèle, pour tous les protocoles. Les rendements de couplage covalent et de couplage passif ont été évalués selon la méthode décrite en c).

*Protocoles de l'art antérieur:*

- protocole "Bangs", Bangs Laboratories Inc., Tech note #13c, Covalent coupling protocols, page 3.
   Les billes ont été lavées dans un tampon de pré-activation (tampon phosphate 50 mM, pH 4,5), puis activées par une solution aqueuse d'EDC en présence d'un faible pourcentage (20 %) de tampon de pré-activation. Le couplage a été réalisé en tampon borate 0,2 M, pH 8,5. Après couplage, les fonctions carboxyliques n'ayant pas réagi ont été bloquées par une solution d'éthanolamine. Les billes ont été conservées dans un tampon contenant de la BSA, de la glycine, du détergent Tween[R] et de l'azoture de sodium.

- protocole J. Sackrison, Covalent coupling to latex particles and diagnostic development using microspheres, the latex course, 1997.
   Les billes ont été lavées plusieurs fois par une solution de tampon borate 10 mM, pH 8,5 ; par une solution d'acétate de sodium 10 mM, pH 5,0 ; et par une solution de diéthanolamine 50 mM, pH 10,2, puis activées par une solution de CMC en tampon diéthanolamine 50 mM, pH 10,2. Le couplage a été réalisé en tampon phosphate 100 mM, NaCl 150 mM, pH 7,4.

- protocole 1, décrit par D.Bastos-Gonzalez et al. J. of colloid and interface science 176, 232-239, 1995.

On a ajouté des billes de latex à une solution tampon (MES) de pH 5,6. Le couplage a été réalisé en milieu faiblement acide (en tampon MES, pH 5,6). Une solution aqueuse d'EDC a été ajoutée, puis l'échantillon a été mis à incuber à température ambiante. Après couplage, les fonctions carboxyliques en excès ont été bloquées par un traitement avec de l'éthanolamine.

Les résultats obtenus par dosage HPLC sont indiqués dans le tableau I sous forme de rendements (%) de couplage et différence (Δ) entre couplage covalent et adsorption passive :

Tableau I

| Protocole | % de peptide couplé passivement | % de peptide couplé par covalence | Δ (covalent - passif) |
|---|---|---|---|
| Bangs | 47 | 57 | 10 |
| Latex course (J. Sackrison) | 80 | 82 | 2 |
| Protocole 1 (D. Bastos-Gonzalez et al.) | 8 | 13 | 5 |
| Protocole selon l'invention | 54 | 100 | 46 |

Les meilleures performances sont obtenues avec le protocole de couplage selon l'invention, en comparaison avec celles obtenues avec les protocoles de l'art antérieur ; ainsi avec le protocole de couplage selon l'invention, le rendement de couplage covalent est quantitatif (100 %) et la différence entre couplage covalent et couplage passif est notable, contrairement aux protocoles de l'art antérieur où la différence entre les 2 formes de couplage est peu significative.

e) Reproductibilité du procédé de couplage selon l'invention

La reproductibilité du procédé de couplage selon l'invention a été étudiée par la réalisation, d'une part, de 3 couplages sur un même lot de billes, et, d'autre part, d'un couplage sur un lot de billes différent.

Un couplage covalent selon le protocole décrit dans la section b.1 (ci-dessus) et un couplage de type "passif" selon le protocole décrit dans la section b.2 (ci-dessus) ont été réalisés en parallèle à chaque expérience. Le calcul du rendement de couplage et l'évaluation du couplage passif ont été déterminés selon le protocole c) ci-dessus.

Les résultats obtenus par dosage HPLC sont indiqués dans le tableau II sous forme de rendements (%) de couplage et différence ($\Delta$) entre couplage covalent et adsorption passive :

Tableau II

| Lot de Billes | % de peptide couplé passivement | % de peptide couplé par covalence | $\Delta$ (covalent - passif) |
|---|---|---|---|
| 477 | 52 | 98 | 46 |
| 477 | 54 | 100 | 46 |
| 477 | 58 | 100 | 42 |
| 583 | 56 | 93 | 37 |

Ces résultats montrent qu'avec un même lot (lot 477), la reproductibilité est excellente. Avec un lot différent (lot 583), le rendement de couplage est tout à fait acceptable, et comparable au rendement obtenu avec le premier lot.

Exemple 2

a) Couplage de sérumalbumine bovine (BSA) et de billes magnétiques carboxyliques

175 µg de BSA (Pantex) sont couplés selon les conditions décrites pour l'exemple 1, à la section b1). La réaction de couplage proprement dite est réalisée pendant 22 heures (au lieu d'1 heure) à température ambiante.

Une adsorption passive est réalisée selon les mêmes conditions en omettant les agents de couplage, CMC et de co-activation, s-NHS. Après couplage, les billes sont séparées par aimantation et le surnageant est conservé pour un dosage en HPLC.

b) Comparaison du couplage de la BSA selon l'invention et de couplages de l'art antérieur

Le protocole selon l'invention a été comparé à 2 protocoles de l'art antérieur : protocole "Bangs" et protocole "latex course" (J. Sackrison).

Les rendements de couplage covalent et d'adsorption passive à l'aide des 3 protocoles ont été évalués selon la méthode décrite dans l'exemple 1, au paragraphe c).

Les résultats obtenus par dosage HPLC sont indiqués dans le tableau III sous forme de rendements (%) de couplage et de différence ($\Delta$) entre couplage covalent et adsorption passive :

Tableau III

| Protocole | % BSA couplée passivement | % BSA couplée par covalence | $\Delta$ (covalent - passif) |
|---|---|---|---|
| Bangs | 13 | 17 | 4 |
| Latex course (J. Sackrison) | 31 | 34 | 3 |
| Protocole selon l'invention | 25 | 65 | 40 |

Les résultats montrent la supériorité du protocole de l'invention par rapport aux protocoles de l'art antérieur, puisqu'il pemiet l'obtention d'un $\Delta$ de couplage (covalent - passif) beaucoup plus élevé.

Exemple 3

Application à un test diagnostique de détection des anticorps anti virus VIH

Les billes obtenues selon le procédé de l'invention, comme cela est indiqué ci-après, ont été utilisées pour la détection d'anticorps anti virus VIH dans un test ELISA connu : test Access[R] HIV 1-2 New, commercialisé par, et disponible chez, Bio-Rad Laboratories, Marnes la Coquette, France, numéro de catalogue : 34 020, dans lequel un anticorps spécifique anti-VIH-2 est pris en sandwich entre un antigène de capture immobilisé sur des billes magnétiques et un antigène marqué par une enzyme. La révélation et la mesure du signal sont réalisées par addition d'un substrat chimioluminescent de l'enzyme et lecture de la luminescence générée.

a) Matériels et méthodes

a1) Antigène de capture (peptide)

Un peptide de 27 AA contenant l'épitope immunodominant essentiel, l'heptapeptide CAFRQVC de la gp36 du virus VIH-2 a été synthétisé selon la méthode de E. Atherton et R.L. Sheppard mentionnée précédemment, puis couplé à la BSA par un couplage covalent à l'aide d'un réactif homobifonctionnel : le bis-(sulfosuccinimyl)subérate. Le conjugué BSA-peptide VIH-2 obtenu est ci-après désigné "BSA-VIH-2".

a2) Immobilisation de fantigène de capture

Le conjugué BSA-VIH-2 ci-dessus a ensuite été couplé à des billes de latex carboxyliques magnétiques (Estapor) selon le protocole de couplage covalent de l'invention et selon un protocole de l'art antérieur

- 12 µg de conjugué BSA-VIH-2 ont été couplés à 100 µl de billes selon la méthode décrite à l'exemple 1b).
- 12 µg de conjugué BSA-VIH-2 ont été couplés à 100 µl de billes selon une méthode de l'art antérieur (protocole 1 de Bastos-Gonzalez : voir exemple 1d).

Les billes magnétiques porteuses de BSA-peptide VIH-2 obtenues sont ci-après désignées "billes BSA-VIH-2".

a3) Antigène de révélation

Le même peptide de 27 AA contenant l'épitope immunodominant essentiel, l'heptapeptide CAFRQVC de la gp36 du virus VIH-2 utilisé en a1) ci-dessus, a été couplé à la phosphatase alcaline (ci-après désignée "PAL"), de la société Biozyme, par couplage covalent à l'aide d'un réactif homobifonctionnel : le bis-(sulfosuccinimyl)subérate. Le conjugué peptide VIH-2-PAL obtenu est ci après désigné "peptide-PAL".

a4) Détection du signal

Un substrat, spécifique de la phosphatase alcaline, et à base de dioxétane, est utilisé pour la révélation. La lecture du signal se fait dans le luminomètre Access[R], disponible chez Bio-Rad Laboratories. France. Le signal est exprimé en unités RLU (Relative Luminescence Units/unités de luminescence relative).

a5) Echantillons

Des sérums humains positifs en anticorps anti-VIH-2 dilués en sérum humain négatif : qc1, qc2, qc3, et un sérum négatif en anticorps anti-VIH : C0, ont été utilisés pour l'essai.

b) Protocole de l'essai

Deux séries en parallèle ont été réalisées.

b1) 50 µl de sérum ont été mis en présence de 50 µg de billes BSA-VIH-2 obtenues selon le protocole de couplage covalent de l'invention, et de 350 µl de conjugué peptide-PAL. Le mélange a été mis à incuber pendant 20 minutes à 37°C. puis les billes ont été séparées par aimantation et le surnageant a été éliminé.
   200 µl de substrat ont été ajoutés et mis à incuber pendant 5 minutes à 37°C.
   La lecture a été réalisée et les RLU enregistrées. Les résultats sont exprimés par le rapport de RLU "Signal/ C0" : voir le Tableau IV.

b2) 50 µl de sérum ont été mis en présence de 50 µg de billes BSA-VIH-2, obtenues selon le protocole 1 de Bastos-Gonzalez, et de 350 µl de conjugué peptide-PAL.

Le reste du protocole est identique à celui du mode opératoire b1) ci-dessus.

c) Résultats et comparaison avec l'art antérieur

Les résultats obtenus avec le couplage selon l'invention et ceux obtenus avec le couplage selon le protocole 1 sont indiqués dans le tableau IV

Tableau IV

| Sérum testé | Protocole 1 | | Protocole selon l'invention | |
|---|---|---|---|---|
| | RLU | SIGNAL/C0 | RLU | SIGNAL/C0 |
| C0 | 15 205 | 0.98 | 10 767 | 1,00 |
| | 15 741 | 1.02 | 10 771 | 1,00 |
| moyenne | 15 473 | | 10 769 | |
| qc1 | 171 048 | 11.05 | 1 894 150 | 175,89 |
| | 168 968 | 10.92 | 1 904 600 | 176,86 |
| qc2 | 15 517 | 1.00 | 181 888 | 16,89 |
| | 15 444 | 1.00 | 183 826 | 17,07 |
| qc3 | 12 731 | 0.82 | 80 437 | 7,47 |
| | 12 591 | 0.81 | 80 462 | 7,47 |

Les résultats montrent, pour les échantillons positifs, un rapport "Signal/C0" obtenu par le protocole selon l'invention considérablement supérieur à celui obtenu par le protocole 1 de l'art antérieur. Ceci se traduit par une bien meilleure immunoréactivité et une meilleure sensibilité analytique, et reflète très clairement que la méthode de couplage selon l'invention a permis un couplage covalent optimum par rapport au protocole 1.

Exemple 4

Essais de stabilité des billes de latex carboxylique magnétique revêtues du conjugué BSA-VIH-2 ("billes BSA-VIH-2") de l'invention.
Une étude de stabilité à long terme : 7 mois, 12 mois et 18 mois à + 4°C, a été réalisée à l'aide du protocole de l'exemple 3 pour des billes BSA-VIH-2 (lot C7P184A, obtenu dans l'exemple 3, à partir de billes du lot initial 477 vu plus haut). Le tableau V résume les résultats obtenus qui montrent que le lot n'a pas perdu significativement d'immunoréactivité en 18 mois à + 4°C. Dans les résultats qui suivent, et pour la commodité de présentation du Tableau V, l'expression « SIGNAL/C0 » a été remplacée par la forme abrégée « S/C0 ».

Tableau V

| Sérum testé | T0 | | T = 7 mois | | T = 12 mois | | T = 18 mois | |
|---|---|---|---|---|---|---|---|---|
| | RLU | S/C0 | RLU | S/C0 | RLU | S/C0 | RLU | S/C0 |
| C0 | 12 144 | | 13 962 | | 12 898 | | 12 768 | |
| | 11 866 | | 13 692 | | 13 855 | | 12 773 | |
| | | | | | 13 586 | | | |
| moyenne | 12 005 | 1.00 | 13 827 | 1.00 | 13 446 | 1.00 | 12 771 | 1.00 |
| qc2 | 139 029 | | 184 633 | | 170 026 | | 182 311 | |
| | 141 796 | | 185 985 | | 173 015 | | 182 952 | |
| | | | | | 173 033 | | | |
| moyenne | 140 413 | 12 | 185 309 | 13 | 172 025 | 13 | 182 632 | 14 |
| qc1 | 1 732 230 | | 2 166 040 | | 2 159 050 | | 2 076 950 | |
| | 1 799 880 | | 2 142 940 | | 2 279 010 | | 2 127 930 | |
| | | | | | 2 102 220 | | | |
| moyenne | 1 766 055 | 147 | 2 154 490 | 156 | 2 180 093 | 162 | 2 102 440 | 165 |

Ces résultats montrent clairement que le procédé selon l'invention permet d'obtenir des complexes de réactifs affins immobilisés - sur phase solide hydrophobe fonctionnalisée par un groupe carboxyle - de grande stabilité dans le temps.

Exemple 5

Couplage covalent d'acide nucléique à des billes de latex carboxyliques magnétiques.

a) matériel et méthodes

Des tampons 5xSSC et 2xSSC sont préparés selon la méthode décrite dans Maniatis T. et al., Molecular Cloning. A laboratory manual, Cold Spring Harbor laboratory, New York (1982).

Des sondes (ADN) fonctionnalisées par une fonction amine en 5' sont obtenues par l'intermédiaire d'un synthétiseur automatique et l'utilisation du réactif commercial aminolink 2 de la Société Perkin Elmer, citées ci-après : sondes 5'-NH$_2$.

L'analyte utilisé est soit de l'ARN, soit de l'ADN. Les performances du couplage de sondes sont évaluées dans un format d'hybridation sandwich sur l'appareil Access[R] de la Société Beckman.

Une sonde ADN spécifique de l'analyte est couplée à des particules magnétiques selon le protocole décrit ci-après en b). Elle sert à capturer l'analyte (sonde de capture).

La révélation est réalisée à l'aide d'une sonde de détection spécifique (différente de la première sonde) de l'analyte marqué, par exemple avec l'enzyme phosphatase alcaline, selon des procédés connus de l'homme du métier.

Des exemples de marquage non radioactif de sondes sont décrits, par exemple, dans le brevet FR n° 78.10975, ou par M.S. Urdea *et al.*, Nucleic Acids Symp. Ser., 24, 1991, 197-200, ou encore par R. Sanchez-Pescador, J. Clin. Microbiol. 26,1988,1934-1938.

Le rendement de couplage covalent d'acide nucléique à des billes de latex carboxyliques magnétiques est évalué comme dans l'exemple 1 par HPLC en utilisant des conditions adaptées aux acides nucléiques : par exemple, phase stationnaire (C18) et phase mobile (gradient de tampon A : acétate de triéthylammonium 10$^{-2}$ M , et tampon B : acétonitrile / A : 95/5).

b) Couplage covalent d'une sonde 5'-NH$_2$ à des billes de latex carboxylique magnétique

20 μg de sonde 5'-NH$_2$ (i.e. porteuse de fonction NH$_2$ en position 5') sont couplés à 200 μl de billes de latex carboxylique magnétique Estapor M1-070/60 selon les conditions similaires à l'exemple b.1.

Ensuite, les billes sont lavées 2 fois avec 500 μl de tampon 5xSSC, 2 fois avec 500 μl de tampon 2xSSC. Les billes sont conservées dans du tampon 2xSSC NaN3 à 0,02 %.

Essai d'hybridation : l'hybridation des sondes de capture et de détection sur l'analyte peut se faire séparément (en deux étapes) ou simultanément (en une étape), notamment selon l'une des méthodes décrites par Langhale et Malcolm, Gene 36, 1985, 201-210 ou par Ranki *et al,* Gene 21, 1993, 77-85, par Dunn et Hassel, Cell, 12, 1977, 23-36 ou encore par Ranki et Soderlund dans les brevets US 4 486 539 et US 4 563 419.

L'homme du métier sera à même de reproduire sans difficulté des expériences d'hybridation et de comparer la méthode de couplage selon l'invention à des techniques de couplage de l'art antérieur. On citera comme techniques de couplage de l'art antérieur la fixation de la sonde de capture sur le support solide selon des procédés bien connus, notamment par adsorption passive ou par couplage covalent (Cook *et al.*, Nucleic Acids Res. 16, 1988, 4077-4095 : Nagata *et al.*, FEBS Lett. 183, 1985, 379-382 : M. Longlaru *et al.*, EP 420 260 A2 ; T. Gingeras *et al.*, EP 276 302 ; E. Kornes et L.M. Kornes, EP 446 260).

**Revendications**

**1.** Procédé d'immobilisation d'un réactif affin sur une phase solide hydrophobe fonctionnalisée par un groupe carboxyle comprenant une étape d'activation de ladite phase solide et une étape de couplage du réactif affin sur ladite phase solide, **caractérisé en ce que** l'on utilise, lors de l'étape d'activation de ladite phase solide, une combinaison d'un carbodiimide et d'un tampon phosphate, en présence d'un coactivateur et en milieu acide ayant un pH allant d'environ 4 à environ 6,5, et **en ce que** l'étape de couplage est réalisée en milieu basique ayant un pH allant d'environ 7,2 à environ 10,5.

**2.** Procédé selon la revendication 1, **caractérisé en ce que** l'on utilise comme carbodiimide un composé choisi dans le groupe comprenant le CMC (N-cyclohexyl-N'-(2-mopholinoéthyl)-carbodiimide méthyl-p-toluène sulfonate) et l'EDC (1-éthyl-3-[3-diméthylaminopropyl]carbodiimide).

**3.** Procédé selon l'une des revendications 1 ou 2, **caractérisé en ce que** l'on utilise comme coactivateur un composé choisi dans le groupe comprenant le s-NHS (sulfo N-hydroxysuccinimide), l'HOBt (1-hydroxybenzotriazole) et le N-hydroxysuccinimide.

**4.** Procédé selon l'une quelconque des revendications 1 à 3, **caractérisé en ce que** l'on utilise comme carbodiimide le CMC (N-cyclohexyl-N'-(2-mopholinoéthyl)-carbodiimide méthyl-p-toluène sulfonate) et comme coactivateur le s-NHS (sulfo-N-hydroxysuccinimide).

**5.** Procédé selon l'une quelconque des revendications 1 à 4, **caractérisé en ce que** le carbodiimide est utilisé à raison de 20 à 50 équivalents molaires par fonction COOH.

**6.** Procédé selon l'une quelconque des revendications 1 à 4, **caractérisé en ce que** le coactivateur est utilisé à raison de 3 à 10 équivalents molaires par fonction COOH.

**7.** Procédé selon l'une quelconque des revendications 1 à 6, **caractérisé en ce que** l'on utilise, lors de l'étape d'activation de la phase solide, une combinaison de 20 à 50 équivalents molaires par fonction COOH de CMC, en tampon phosphate de 30-200 mM, en présence de 3 à 10 équivalents molaires par fonction COOH de coactivateur sulfo-N-hydroxysuccinimide, en milieu acide ayant un pH allant d'environ 4 à environ 6,5 et **en ce que** l'on réalise le couplage en milieu basique ayant un pH allant d'environ 7,2 à environ 10,5.

**8.** Procédé selon l'une quelconque des revendications 1 à 6, **caractérisé en ce que** l'on utilise lors de l'étape d'activation de la phase solide, une combinaison de 30 équivalents molaires par fonction COOH de CMC, en tampon phosphate $KH_2PO_4$ 50 mM, en présence de 5 équivalents molaires par fonction COOH de coactivateur sulfo-N-hydroxysuccinimide, à pH 6, et l'on réalise le couplage dans un milieu contenant un volume d'un tampon de pH 8,5 et un volume dudit tampon phosphate.

**9.** Procédé selon l'une quelconque des revendications 1 à 8, **caractérisé en ce que** le réactif affin contient une fonction amine ou peut en être artificiellement pourvu.

**10.** Procédé selon la revendication 9, **caractérisé en ce que** le réactif affin est choisi dans le groupe comprenant les protéines, les peptides, les immunoglobulines, les antigènes, les haptènes, les anticorps, les enzymes, les substrats enzymatiques, les oligonucléotides, et les polynucléotides.

**Patentansprüche**

**1.** Verfahren zur Immobilisierung eines affinen Reaktanten auf einer hydrophoben Festphase, die mit einer Carboxygruppe funktionalisiert ist, das einen Schritt der Aktivierung der Festphase und einen Schritt der Kupplung des affinen Reaktanten an der Festphase umfasst, **dadurch gekennzeichnet, dass** bei der Aktivierung der Festphase eine Kombination aus einem Carbodiimid und einem Phosphatpuffer in Gegenwart eines Coaktivators in einem sauren Medium mit einem pH-Wert von etwa 4 bis etwa 6,5 verwendet wird und dadurch, dass der Kupplungsschritt in einem basischen Medium mit einem pH-Wert von etwa 7,2 bis etwa 10,5 durchgeführt wird.

**2.** Verfahren nach Anspruch 1, **dadurch gekennzeichnet, dass** als Carbodiimid eine Verbindung verwendet wird, die unter CMC (N-Cyclohexyl-N'-(2-morpholinoethyl)-carbodiimid-methyl-p-toluolsulfonat) und EDC (1-Ethyl-3-[3-dimethylaminopropyl]-carbodiimid) ausgewählt ist.

**3.** Verfahren nach einem der Ansprüche 1 oder 2, **dadurch gekennzeichnet, dass** als Coaktivator eine Verbindung verwendet wird, die unter s-NHS (Sulfo-N-hydroxysuccinimid), HOBt (1-Hydroxybenzotriazol) und N-Hydroxysuccinimid ausgewählt ist.

**4.** Verfahren nach einem der Ansprüche 1 bis 3, **dadurch gekennzeichnet, dass** als Carbodiimid das CMC (N-Cyclohexyl-N'-(2-morpholinoethyl)-carbodiimid-methyl-p-toluolsulfonat und als Coaktivator das s-NHS (Sulfo-N-hydroxysuccinimid) verwendet wird.

**5.** Verfahren nach einem der Ansprüche 1 bis 4, **dadurch gekennzeichnet, dass** das Carbodiimid in einer Menge von 20 bis 50 Moläquivalenten pro COOH-Funktion verwendet wird.

**6.** Verfahren nach einem der Ansprüche 1 bis 4, **dadurch gekennzeichnet, dass** der Coaktivator in einer Menge von 3 bis 10 Moläquivalenten pro COOH-Funktion verwendet wird.

7. Verfahren nach einem der Ansprüche 1 bis 6, **dadurch gekennzeichnet, dass** bei der Aktivierung der Festphase eine Kombination aus 20 bis 50 Moläquivalenten CMC pro COOH-Funktion in Phosphatpuffer von 30 bis 200 mM in Gegenwart von 3 bis 10 Moläquivalenten Coaktivator Sulfo-N-hydroxysuccinimid pro COOH-Funktion in einem sauren Medium mit einem pH-Wert von etwa 4 bis etwa 6,5 verwendet wird, und dadurch, dass die Kupplung in einem basischen Medium erfolgt, das einen pH-Wert von etwa 7,2 bis etwa 10,5 aufweist.

8. Verfahren nach einem der Ansprüche 1 bis 6, **dadurch gekennzeichnet, dass** bei der Aktivierung der Festphase eine Kombination aus 30 Moläquivalenten CMC pro COOH-Funktion in $KH_2PO_4$-Phosphatpuffer 50 mM in Gegenwart von 5 Moläquivalenten Coaktivator Sulfo-N-hydroxysuccinimid pro COOH-Funktion bei pH 6 verwendet wird und die Kupplung in einem Medium erfolgt, das ein Volumen eines Puffers von pH 8,5 und ein Volumen des Phosphatpuffers enthält.

9. Verfahren nach einem der Ansprüche 1 bis 8, **dadurch gekennzeichnet, dass** der affine Reaktant eine Aminofunktion aufweist oder nachträglich mit einer Aminofunktion versehen werden kann.

10. Verfahren nach Anspruch 9, **dadurch gekennzeichnet, dass** der affine Reaktant unter den Proteinen, Peptiden, Immunglobulinen, Antigenen, Haptenen, Antikörpern, Enzymen, enzymatischen Substraten, Oligonucleotiden und Polynucleotiden ausgewählt ist.

**Claims**

1. Method of immobilizing an affinity reagent on a hydrophobic solid phase functionalized by a carboxyl group, said method comprising a step for activation of said solid phase and a step for coupling of the affinity reagent to said solid phase, **characterized in that** the step for activation of said solid phase uses a combination of a carbodiimide and a phosphate buffer in the presence of a co-activator and in an acid medium with a pH ranging from about 4 to about 6.5, and **in that** the coupling step is performed in a basic medium with a pH ranging from about 7.2 to about 10.5.

2. Method according to claim 1, **characterized in that** the carbodiimide used is a compound selected from the group comprising CMC (N-cyclohexyl-N'-(2-morpholinoethyl)carbodiimide methyl-p-toluenesulfonate) and EDC (1-ethyl-3-[3-dimethylaminopropyl]carbodiimide).

3. Method according to claim 1 or 2, **characterized in that** the co-activator used is a compound selected from the group comprising s-NHS (sulfo-N-hydroxysuccinimide), HOBt (1-hydroxybenzotriazole) and N-hydroxysuccinimide.

4. Method according to any one of claims 1 to 3, **characterized in that** the carbodiimide used is CMC (N-cyclohexyl-N'-(2-morpholinoethyl)carbodiimide methyl-p-toluenesulfonate) and the co-activator used is s-NHS (sulfo-N-hydroxysuccinimide).

5. Method according to any one of claims 1 to 4, **characterized in that** the carbodiimide is used in an amount of 20 to 50 molar equivalents per COOH group.

6. Method according to any one of claims 1 to 4, **characterized in that** the co-activator is used in an amount of 3 to 10 molar equivalents per COOH group.

7. Method according to any one of claims 1 to 6, **characterized in that** the step for activation of the solid phase uses a combination of 20 to 50 molar equivalents per COOH group of CMC, in 30-200 mM phosphate buffer, in the presence of 3 to 10 molar equivalents per COOH group of sulfo-N-hydroxysuccinimide co-activator, in an acid medium with a pH ranging from about 4 to about 6.5, and **in that** the coupling is performed in a basic medium with a pH ranging from about 7.2 to about 10.5.

8. Method according to any one of claims 1 to 6, **characterized in that** the step for activation of the solid phase uses a combination of 30 molar equivalents per COOH group of CMC, in 50 mM $KH_2PO_4$ phosphate buffer, in the presence of 5 molar equivalents per COOH group of sulfo-N-hydroxysuccinimide co-activator, at pH 6, and the coupling is performed in a medium containing 50% of a buffer of pH 8.5 and 50 % of said phosphate buffer.

9. Method according to any one of claims 1 to 8, **characterized in that** the affinity reagent contains an amine group or can be artificially provided with an amine group.

10. Method according to claim 9, **characterized in that** the affinity reagent is selected from the group comprising proteins, peptides, immunoglobulins, antigens, haptens, antibodies, enzymes, enzyme substrates, oligonucleotides and polynucleotides.

Solution témoin àT$_0$

FIG.1

Couplage covalent à T$_{1h}$

FIG.2

Couplage passif à T$_{1h}$

FIG.3